Europäisches Patentamt

European Patent Office

Office européen des brevets

(19)

(11) Numéro de publication: **0 148 668**
**B1**

(12) **FASCICULE DE BREVET EUROPÉEN**

(45) Date de publication du fascicule du brevet:
13.09.89

(21) Numéro de dépôt: **84402482.8**

(22) Date de dépôt: **04.12.84**

(51) Int. Cl.⁴: **C 12 N 15/00,** C 12 P 21/02,
C 12 N 1/16 //
(C12N1/16, C12R1:865)

(54) Vecteurs plasmidiques de clonage et d'expression d'une protéine dans un micro-organisme, comportant au moins le promoteur d'expression de la beta-glucosidase dans les levures; micro-organismes les contenant; procédé de fermentation et enzymes obtenues.

(30) Priorité: **06.12.83 FR 8319494**

(43) Date de publication de la demande:
**17.07.85 Bulletin 85/29**

(45) Mention de la délivrance du brevet:
**13.09.89 Bulletin 89/37**

(84) Etats contractants désignés:
**AT BE CH DE FR GB IT LI LU NL SE**

(56) Documents cité:
**FR-A-2 489 363**

**BIOLOGICAL ABSTRACTS, vol. 72, no. 8, 1981, page 5388, no. 51899, Philadelphia, US; R.W. ARMENTROUT et al.: "Molecular cloning of genes for cellobiose utilization and their expression in Escherichia coli" & APPL. ENVIRON. MICROBIOL. 41(6): 1355-1362, 1981**
**BIOLOGICAL ABSTRACTS, RRM, no. 21003547, Philadelphia,.US; S.K. PICATAGGIO et al.: "The cloning of trichoderma-reeseigenomic DNA in Escherichia-coli HB-101" & ABSTRACTS OF THE ANNUAL MEETING OF THE AMERICAN SOCIETY FOR MICROBIOLOGY (USA) 1981, vol. 81, no. P116**

(73) Titulaire: **Etablissement Public dit: CENTRE NATIONAL DE LA RECHERCHE SCIENTIFIQUE (CNRS), 15, Quai Anatole France, F-75007 Paris (FR)**

(72) Inventeur: **Guerineau, Michel, 45, rue St Placide, F-75006 Paris (FR)**
Inventeur: **Raynal, Alain, 68, rue St Nicolas, F-91940 Gomez Lechatel (FR)**

(74) Mandataire: **Warcoin, Jacques, Cabinet Régimbeau 26, avenue Kléber, F-75116 Paris (FR)**

**Description**

La présente invention concerne, de façon générale, des vecteurs plasmidiques de clonage et d'expression d'une protéine dans un micro-organisme, et en particulier dans les levures, les micro-organismes transformés par lesdits vecteurs plasmidiques, un procédé de fermentation et les enzymes obtenues par la mise en oeuvre dudit procédé.

Plus particulièrement, la présente invention concerne des vecteurs plasmidiques de clonage et d'expression du gène de la β-glucosidase dans les levures, ainsi que son application à la dégradation enzymatique des matières cellulosiques.

La dégradation des matières cellulosiques correspond, en effet, à une préoccupation économique actuelle de premier ordre. La cellulose est un des constituants principaux du bois, des céréales et de toute substance végétale en général. La valorisation des sources naturelles et renouvelables que constituent les sous-produits de la sylviculture, l'agriculture, l'élevage et des industries agro-alimentaires entre-autres, par la dégradation de la cellulose en glucose, est une voie importante de production d'énergie, mais également de synthèse de produits tels que: des protéines, des enzymes, des antibiotiques et des intermédiaires de chimie organique (solvants, acides organiques).

Le récent développement de la technologie des enzymes a conduit à s'intéresser à l'hydrolyse enzymatique de la cellulose qui pourrait se révéler plus rentable que les procédés chimiques utilisés jusqu'alors. On sait que la β-glucosidase (EC3-2-1-21) intervient dans la dernière étape de la dégradation de la cellulose. Elle permet de scinder le cellobiose en deux molécules de glucose.

Nombre de bactéries et de champignons produisent des cellulases utilisées industriellement. Toutefois, l'hydrolyse de la cellulase par le complexe cellulosique se traduit par une accumulation de cellobiose qui inhibe l'activité cellulosique. La vitesse d'hydrolyse de la cellulose pourrait donc être largement augmentée en complétant l'action des cellulases par des β-glucosidases immobilisées. Cette enzyme immobilisée montre une grande stabilité. De plus elle peut être réutilisée.

Un autre intérêt serait de se doter d'un organisme capable directement de fermenter le cellobiose. Dans cette optique, la levure semble être un organisme de chois. En effet, la levure est cultivée industriellement sur des milieux à bas prix de revient pour la production de levure de panification de bière. Elle est consommée par l'homme depuis des millénaires, elle ne peut se multiplier chez l'homme et ne possède aucun pouvoir pathogène. C'est donc un hôte très intéressant pour les recombinaisons génétiques in vitro, en vue de la sélection de nouvelles souches industrielles ou la production d'enzymes, ou de protéines par exemple.

Le clonage d'un gène de structure de la β-glucosidase dans <u>Saccharomyces cerevisiae</u> a permis d'obtenir des transformants de <u>S. cerevisiae</u> produisant la β-glucosidase à un taux élevé. L'étude de la structure du gène cloné à révélé que ce gène est transcrit à partir d'un promoteur fort, c'est-à-dire que le promoteur d'expression de la β-glucosidase dans les levures, peut être utilisé à la production à un taux élevé de protéines - autres que la β-glucosidase - par fusion entre ce promoteur et le gène de structure correspondant ou le cDNA synthétisé à partir de l'ADN messager mature, que l'on désire cloner.

Un autre avantage de la présente invention réside donc dans l'isolement et le sous-clonage du fragment d'ADN portant ce promoteur fort.

C'est pourquoi la présente invention concerne des vecteurs plasmidiques de clonage et d'expression d'une protéine dans un micro-organisme, caractérisés en ce qu'ils comportent au moins le gène de structure codant pour la synthèse de ladite protéine et les éléments assurant l'expression dudit gène de structure dans un micro-organisme, et en ce que la promotion du gène de structure est assurée par le promoteur d'expression du gène de la β-glucosidase dans les levures.

Selon un mode de réalisation de l'invention, le promoteur d'expression du gène de la β-glucosidase dans les levures consiste en une séquence d'ADN chromosomique extrait d'une levure, notamment d'une souche de <u>Kluyveromyces fragilis</u>. Il s'agira, en particulier, de tout ou partie du fragment de restriction BamH1-BamH1 de 2,2 Kb sous-cloné à partir d'un vecteur plasmidique selon l'invention.

Selon un autre mode de réalisation de l'invention, le gène de structure de la protéine que l'on désire cloner, est, de préférence, un gène eucaryote, en particulier le gène codant pour la synthèse de β-glucosidase. Ce gène proviendra, notamment, de l'ADN chromosomique d'une souche de <u>K. fragilis</u> produisant une β-glucosidase de manière constitutive et constituant ainsi l'organisme donneur.

On entend par "éléments assurant l'expression du gène de structure" toutes les séquences d'ADN nécessaires à cette expression dans un micro-organisme, excepté le gène de structure lui-même de la protéine que l'on désire cloner, c'est-à-dire outre le promoteur, un élément de terminaison lorsque cela est nécessaire et/ou leur codon de départ en phase, par exemple.

Selon un mode de réalisation préféré de l'invention, lesdits plasmides comprennent au moins:

- une origine de réplication dans les levures, notamment l'origine de réplication d'un plasmide 2 µm de levure,
- un gène de levure permettant une sélection parmi les levures.

Les vecteurs plasmidiques selon l'invention peuvent comprendre en outre des éléments permettant leur transfert dans les bactéries lorsqu'il s'agit d'un plasmide "navette", notamment:

- une origine de réplication dans les bactéries, par exemple dans <u>Escherichia coli</u>,

- et des gènes codant pour la résistance à certains antibiotiques, notamment à l'Ampicilline et à la Tétracycline.

L'ADN du plasmide 2 μm portant l'origine de réplication dans les levures permet, aux vecteurs plasmidiques selon l'invention, d'être amplifiés dans une levure qui constitue, comme il a été indiqué ci-avant, l'hôte de choix pour les recombinaisons génétiques qui font l'objet de la présente invention.

L'intérêt du gène ura3+ réside dans le fait qu'il permet la sélection des micro-organismes transformants. Après transformation d'une souche de phénotype ura3-, seuls les micro-organismes ayant intégré un vecteur plasmidique selon l'invention se développeront sur un milieu minimum sans uracile. La présence d'une origine de réplication dans les bactéries se justifie car on utilise une bactérie, en particulier Escherichia coli, comme hôte intermédiaire au cours du processus de construction desdits vecteurs tel qu'il sera décrit ci-après.

De même, la présence de gènes codant pour une résistance à certains antibiotiques, Ampicilline et Tétracycline entre autres, permet une sélection des bactéries transformées au cours du processus de construction des vecteurs plasmidiques.

Indépendamment des vecteurs plasmidiques décrits précédemment, la présente invention concerne également les micro-organismes transformés par les vecteurs selon la présente invention.

Compte-tenu d'une part, que l'application principale de l'invention consiste en la préparation de micro-organismes capables de fermenter directement le cellobiose, et que, d'autre part et comme il a été indiqué en introduction, les levures et plus particulièrement celles appartenant au genre Saccharomyces, semblent être les micro-organismes s'adaptant au mieux à la réalisation de cet objectif, l'invention protège les levures transformées par lesdits vecteurs plasmidiques, et de manière plus spécifique une souche transformée de S. cerevisiae.

Enfin, la présente invention concerne un procédé de production de protéines, en particulier de β-glucosidase, consistant à faire fermenter un milieu approprié par un microorganisme, en particulier une levure, comme décrit ci-avant et à isoler la protéine obtenue.

En outre, la présente invention concerne la protéine (la β-glucosidase) obtenue par la mise en oeuvre dudit procédé.

La composition des milieux de fermentation est connue de l'homme de métier, en particulier pour ce qui concerne les fermentation par S. cerevisiae, mais dans certains cas il sera intéressant de pouvoir fermenter directement la cellulose soit en utilisant une souche portant les gènes assurant cette fermentation, et en particulier le gène codant pour la β-glucosidase selon l'invention, soit en utilisant diverses souches dont celles selon l'invention.

L'isolement de la β-glucosidase produite peut être effectué par tout procédé après éclatement éventuel des cellules lorsque l'enzyme n'est pas excrétée.

Les exemples ci-après permettront de mettre en évidence d'autres caractéristiques et avantages de la présente invention.

L'utilisation des enzymes de restriction et des ligases est connue de l'homme de métier et afin d'alléger la description, sauf indication contraire, les enzymes en cause seront mises en oeuvre conformément aux prescriptions du fabricant. Ces enzymes sont commercialisées notamment par la Société BIOLABS, la Société MILES LABORATORIES INC. et la Société BOEHRINGER.

Les figures ci-annexées permettront de mieux comprendre certains aspects de la présente invention.
Sur ces figures:

- la figure 1 représente une carte de restriction du plasmide KF1,
- la figure 2 représente une carte de restriction du plasmide KF4,
- et la figure 3 représente une carte de restriction du plasmide PR1.

## Exemple 1

### Construction d'une banque de gène de Kluyveromyces fragilis dans Escherichia coli.

A: Organisme donneur:

L'organisme donneur est une levure Kluyveromyces fragilis (Y610) déposée à l'ATCC sous le n° 12424. Cette souche produit de manière constitutive une β-glucosidase caractérisée par L.W. FLEMING et J.D. DUERKSEN: Purification and Characterization of Yeast β-glucosidase; Journal of Bacteriology vol. 93 (1967), pp. 135 - 141.

L'ADN de K. fragilis est purifié par centrifugation à l'équilibre en gradient de CsCl. Après lyse des cellules de levure, on ajoute au lysat une solution de CsCl saturée dans les proportions 1/3 et 2/3 respectivement. Le mélange est centrifugé 48 heures à 40 K rpm dans un rotor TFT65 Kontron. Le contenu du tube est collecté par perçage du fond du tube, la présence de l'ADN est révélée par l'écoulement de liquide très visqueux.

Après dialyse de l'ADN collecté contre du TE (10 mM tris, 1 mM EDTA pH 7,5) pendant 24 heures, l'ADN est analysé par électrophorèse en gel d'agarose.

Par électrophorèse sur gel à 0,3 % avec comme étalon de taille l'ADN du phage λ (49 kb), on peut estimer la taille de l'ADN purifié à environ 150 Kb au moins.

B - Vecteur de clonage:

Le vecteur de clonage utilisé est le cosmide pHCG3 décrit par C. GERBAUD et collaborateurs: Construction of new yeast vectors and cloning of the nif (Nitrogen Fixation) gene cluster of Klebsiella pneumoniae in yeast; Current Genetics vol. 3 (1981), pp. 173-180. Ce vecteur est constitué d'une partie du plasmide de 2 μm isolé de S. cerevisiae, portant l'origine de réplication de ce plasmide, et d'un gène de levure ura3+.

L'autre partie de ce vecteur est le cosmide pHC79 décrit par B. HOHN et J. COLLINS: A small cosmid for efficient cloning of large DNA fragments; Gene vol. 10 (1980), pp. 291 - 298. Le cosmide pHCG3 est un vecteur dit "navette", c'est-à-dire qu'il peut se répliquer et être sélectionné à la fois dans S. cerevisiae et dans E. coli, il est décrit notamment dans le brevet français n° 80 18754.

Il possède en outre des propriétés de cosmide, ce qui permet le clonage de fragments d'ADN de grande taille.

C - Clonage du gène de β-glucosidase dans pHCG3

L'ADN de K. fragilis est digéré partiellement par l'enzyme de restriction Sau3A, permettant de couper l'ADN chromosomique de manière "pseudo aléatoire". Après digestion des fragments de 20 à 40 Kb sont purifiés par centrifugation en gradient de saccharose (10 à 40 %) tourné 20 heures à 25 K rpm dans un rotor SW41 Beckman.

Parallèlement, le cosmide pHCG3 est totalement digéré par l'enzyme de restriction BamH1.

Les fragments purifiés de l'ADN de K. fragilis sont ligaturés, par action de la T4 DNA-ligase en présence d'ATP et de dithiottreitol pendant une nuit à 13°C, au cosmide pHCG3 digéré. Les ensymes Sau3A et BamH1 donnent après digestion des extrémités compatibles.

D - Préparation du plasmide KF1

Le mélange de ligation issu de l'étape C est ensuite encapsidé dans des coques vides de bactériophages Lambda selon le protocole de B. HOHN et J. COLLINS. Cosmids: A type of plasmid gene cloning vector that is packageable in vitro in bacteriophage Lambda Heads; Proc. Natl. Acad. Sci. U.S.A. vol. 75 n° 9 (1978), pp. 4242 - 4246.

Le mélange d'encapsidation préparé précédemment est utilisé pour infecter une souche d'E. coli HB101 qui est notamment sensible à l'Ampicilline et à la Tétracycline. Le vecteur utilisé portant les gènes bactériens de résistance à ces deux antibiotiques, on pourra sélectionner les bactéries infectées qui seront résistantes à l'Ampicilline (50 μg/ml). Les insertions d'ADN de K. fragilis sont faites au site BamH1 du vecteur, situé dans le gène de résistance à la Tétracycline. De ce fait, les bactéries ayant reçu un plasmide recombinant portant de l'ADN de K. fragilis seront Ampicilline résistance (AmpR) et Tétracycline sensible (TcS). 1800 clones bactériens AmpR TcS ont ainsi été isolés. Ce chiffre est en théorie suffisant pour être sûr à 98 % d'avoir cloné un gène donné de K. fragilis.

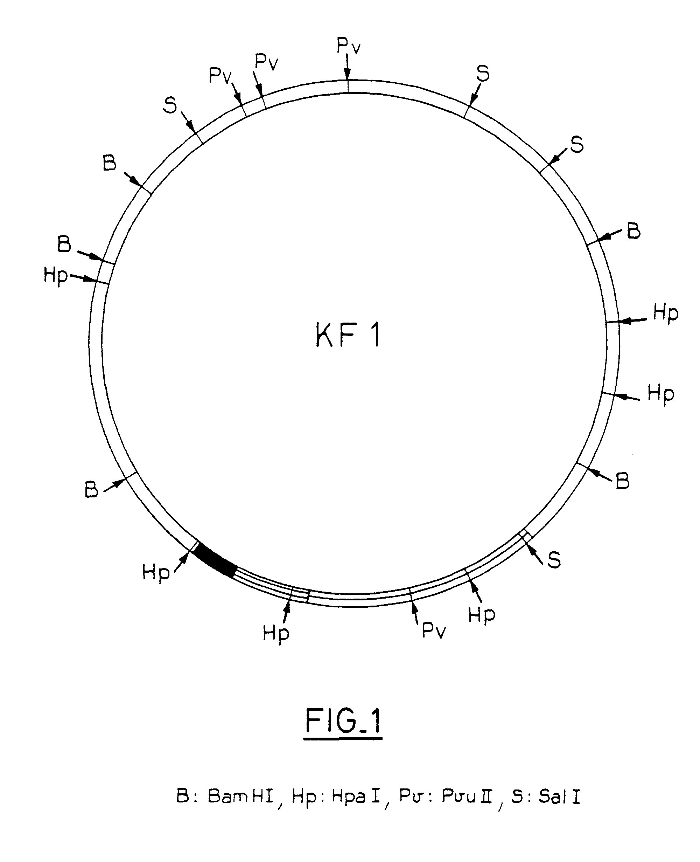

Le plasmide recombinant ainsi préparé est appelé plasmide KF1. Sa structure a été ensuite étudiée (figure 1). Le plasmide KF1 est constitué d'une seule copie du vecteur pHCG3 et d'une insertion d'ADN de K. fragilis d'environ 35 Kb, l'ensemble du plasmide ayant une taille de 45 Kb.

E - Recherche de l'activité β-glucosidase

L'activité β-glucosidase a été recherchée parmi ces 1800 colonies, en dosant la capacité à hydrolyser le PNPG (p-nitrophenyl β-D-glucoside). La coupure de la liaison 1 - 4 β-D-glucoside libère des groupements nitrophénol donnant une coloration jaune quantifiable en densité optique à 400 nm; 1 nM de PNPG hydrolysée produit une augmentation de Do de 0,013. Le test d'activité est fait sur des bactéries préalablement lysées par action de lysozyme et d'un détergent doux le Triton X100. On a isolé ainsi un clone parmi les 1800 ayant l'activité recherchée. L'ADN plasmidique isolé de ce clone a été utilisé pour transformer la souche HB101. Tous les transformants résultants produisaient de la β-glucosidase. On montre ainsi que l'on a cloné sur un plasmide, appelé KF1, un fragment d'ADN portant le gène de structure de la β-glucosidase de K. fragilis.

F - Préparation du plasmide KF4

Cette préparation consiste à réduire, par sous-clonages successifs, la taille du plasmide KF1 et plus précisément de réduire la taille du fragment d'ADN de K. fragilis inséré au cosmide pHCG3 de façon à ne conserver de ce fragment que la partie comportant le gène de structure et la région promotrice de la protéine que l'on désire cloner.

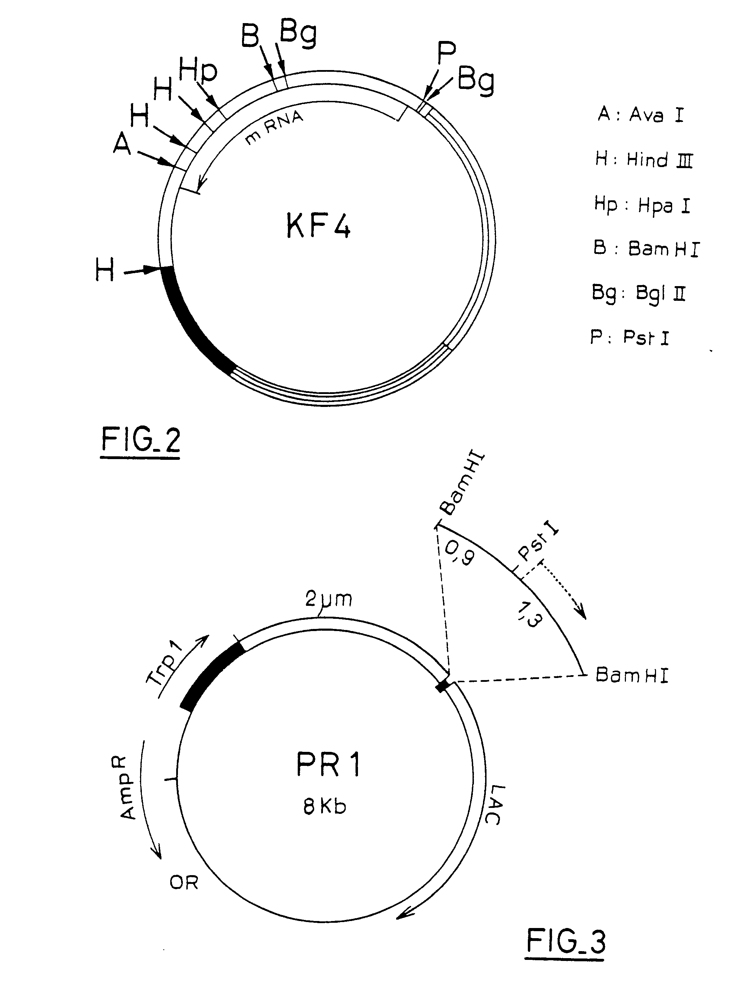

Les sous-clonages consistent à chaque étape à faire des délétions dans l'insertion portée par le plasmide pHCG3. A chaque étape on recherche après transformation de la souche HB101 de E. coli des transformants β-glucosidase+ portant une insertion d'ADN de taille réduite. On est ainsi passé en trois étapes successives du

4

EP 0 148 668 B1

plasmide KF1 portant une insertion de 35 Kb au plasmide KF4 pour lequel la taille de l'insertion a été réduite à 3,5 Kb (figure 2).

## Exemple 2

### Transformation d'une levure par le plasmide KF4

A - Préparation d'une souche de S. cerevisiae transformée TYKF4

Les plasmides sélectionnés dans E. coli ont été, après purification, transférés dans la levure S. cerevisiae par transformation. La souche réceptrice utilisée pour la transformation est la souche OL1, double mutante pour le gène ura3. La souche OL1 de S. cerevisiae ainsi transformée constitue la souche TYKF4.

Les plasmides utilisés portent le gène de levure ura3+, et après transformation on sélectionne des colonies de levure prototrophe sur milieu minimum sans uracile.

B - Expression de la β-glucosidase dans la levure

Les clones sélectionnés ont donc reçu le plasmide et on a étudié l'expression du gène de la β-glucosidase dans les transformants TYKF4 de S. cerevisiae. Les dosages enzymatiques ont été effectués sur des extraits bruts de levure obtenues par broyage avec des billes de verre.

Les essais ont été faits en utilisant, d'une part, le PNPG, d'autre part, le cellobiose comme substrats. Ce test permettant de déterminer le niveau d'expression du gène et de savoir si la β-glucosidase produite était capable d'hydrolyser le cellobiose.

Les résultats obtenus sont présentés dans le tableau 1. On peut noter une activité spécifique, pour les levures transformées par le plasmide KF4, plus de 300 fois supérieure à celle observée pour la souche de K. fragilis.

On considère:

- le turn-over de la β-glucosidase de levure purifiée donnée par H.N. INAMAR et J.C. KAPLAN: $12{,}1 \times 10^3$ molécules de PNPG hydrolysées par minute par molécule d'enzyme.
- un poids moléculaire moyen pour la β-glucosidase de 300 000 daltons.
- l'activité spécifique de OL1 transformée par KF4 (TYKF4) qui est de 3650 nM de PNPG hydrolysées par minute par mg de protéine.
- et l'activité spécifique de OL1 transformée par KF4 (TYKF4) qui est de 600 µg de glucose libéré par minute et par mg de protéine. On précise que ceci correspond à 1700 nM de cellobiose hydrolysées par minute et par mg de protéine.

On peut estimer que dans TYKF4 la β-glucosidase produite représenterait de l'ordre de 10 % des protéines totales.

Ce résultat fait apparaître que le gène de la β-glucosidase serait transcrit à partir d'un promoteur fort.

## Exemple 3

### Sous-clonage de la région promotrice

Après avoir déterminé à partir du plasmide KF4 la taille et le sens de la transcription du gène de la β-glucosidase, il convenait, pour caractériser la région promoteur, de sous-cloner un fragment recouvrant le gène situé en amont du site Pst1 de KF4 (figure 2). On est donc retourné au plasmide KF1 pour sous-cloner le fragment BamH1 - BamH1 de 2,2 Kb recouvrant cette zone (figure 1).

Afin de tester si ce fragment porte le promoteur recherché, on effectue des fusions avec le gène lacZ produisant la β-galactosidase. On utilise le plasmide pMC2010 construit par CASADABAN, qui porte le gène lacZ de l'opéron lactose, dépourvu de région promoteur et de codon d'initiation. Les souches de E. coli et de S. cerevisiae transformées par ce plasmide ont donc un phénotype β-galactosidase-. Si on insère dans ce plasmide un fragment d'ADN portant un promoteur et un codon d'initiation de la transcription on doit alors permettre la production de β-galactosidase. Le fragment BamH1 - BamH1 de 2,2 Kb isolé après digestion de KF1 a été inséré au site BamH1 de pMC2010. On a préparé ainsi le plasmide PR1. Après ligation en utilisant la T4 DNA ligase et transformation d'E. coli, on a obtenu des transformants produisant la β-galactosidase. Le plasmide recombiné isolé de tels transformants a été, après purification, utilisé pour transformer S. cerevisiae. On a pu montrer que les transformants résultants produisaient la β-galactosidase (figure 3). Ces résultats montrent donc clairement que le fragment sous-cloné de 2,2 Kb porte bien la région promotrice.

5

**Tableau 1**

| Vecteur plasmidique | Souche | Activité spécifique testée sur | |
|---|---|---|---|
| | | PNPG | Cellobiose |
| - | OL1 | 1 | 0,22 |
| - | K. fragilis | 7,6 | 2,1 |
| KP4 | TYLF4 | 3636 | 600 |

- L'activité spécifique testée sur le PNPG s'exprime en n Moles hydrolysées /mn/mg de protéine,
- L'activité spécifique testée sur le cellobiose s'exprime en µg de glucose libéré /mn/mg de protéine.

**Revendications**

1. Vecteur plasmidique de clonage et d'expression d'une protéine dans une levure, caractérisé en ce qu'il comporte au moins le gène de structure codant pour la synthèse de ladite protéine et les éléments assurant l'expression dudit gène de structure dans ladite levure, et en ce que la promotion du gène de structure est assurée par le promoteur d'expression du gène de la β-glucosidase, ledit promoteur consistant en une séquence d'ADN chromosomique, extrait d'une souche de Kluyveromyces fragilis, et constitué de tout ou partie du fragment de restriction BamH1 - BamH1 de 2,2 Kb.

2. Vecteur plasmidique selon la revendication 1, caractérisé en ce que le gène de structure code pour la synthèse de β-glucosidase.

3. Vecteur plasmidique selon les revendications 1 et 2, caractérisé en ce que le gène de structure est le gène de Kluyveromyces fragilis codant pour la synthèse de β-glucosidase.

4. Vecteur plasmidique selon l'une des revendications 1 à 3, caractérisé en ce qu'il comprend en outre:
- une origine de réplication dans les levures,
- et un gène assurant une sélection dans les levures.

5. Vecteur plasmidique selon la revendication 4, caractérisé en ce que l'origine de réplication dans les levures est l'origine de réplication du plasmide de 2 µ.

6. Vecteur plasmidique selon la revendication 4, caractérisé en ce que le gène assurant une sélection est le gène ura3+.

7. Vecteur plasmidique selon l'une des revendications 1 à 6, caractérisé en ce qu'il comprend en outre:
- une origine de réplication dans les bactéries, notamment dans Escherichia Coli,
- et des gènes de résistance à certains antibiotiques, notamment à l'Ampicilline et à la Tétracycline.

8. Levure transformée par un vecteur plasmidique selon l'une des revendications 1 à 7.

9. Levure selon la revendication 8, caractérisée en ce qu'elle appartient au genre Saccharomyces.

10. Levure selon la revendication 9, caractérisée en ce qu'il s'agit de Saccharomyces cerevisiae.

11. Procédé de production d'une protéine caractérisé en ce qu'il consiste à faire fermenter un milieu par une levure selon l'une des revendications 8 à 10, et à isoler la protéine produite.

12. Procédé selon la revendication 11, caractérisé en ce que la protéine produite est la β-glucosidase.

13. β-glucosidase obtenue par la mise en oeuvre du procédé selon la revendication 11, au moyen du vecteur plasmidique de la revendication 3.

**Patentansprüche**

1. Klonierungs- und Expressions-Plasmidvektor eines Proteins in einer Hefe, dadurch gekennzeichnet, daß er mindestens das Strukturgen, das für die Synthese dieses Proteins kodiert, und die Elemente, die die Expression dieses Strukturgens in der Hefe sicherstellen, umfaßt, und daß die Promotion des Strukturgens durch den Expressionspromotor des Gens von β-Glucosidase sichergestellt wird, wobei der Promotor aus einer Chromosomen-DNA-sequenz, extrahiert aus einem Stamm von Kluyveromyces fragilis, besteht und ganz oder teilweise aus dem Restriktionsfragment BamH1 - BamH1 von 2,2 Kb aufgebaut ist.

2. Plasmidvektor nach Anspruch 1, dadurch gekennzeichnet, daß das Strukturgen für die Synthese von β-Glucosidase kodiert.

3. Plasmidvektor nach den Ansprüchen 1 und 2, dadurch gekennzeichnet, daß das Strukturgen das Gen von Kluyveromyces fragilis ist, das für die Synthese von β-Glucosidase kodiert.

4. Plasmidvektor nach einem der Ansprüche 1 bis 3, dadurch gekennzeichnet, daß er außerdem umfaßt:
- einen Replikationsursprung in den Hefen,
- ein Gen, das eine Selektion in den Hefen sicherstellt.

5. Plasmidvektor nach Anspruch 4, dadurch gekennzeichnet, daß der Replikationsursprung in den Hefen der Replikationsursprung des Plasmids von 2 µ ist.

6. Plasmidvektor nach Anspruch 4, dadurch gekennzeichnet, daß das Gen, das eine Selektion sicherstellt, das Gen ura3+ ist.

7. Plasmidvektor nach einem der Ansprüche 1 bis 6, dadurch gekennzeichnet, daß er außerdem umfaßt:
- einen Replikationsursprung in den Bakterien, insbesondere in <u>Escherichia Coli,</u>
- und Resistenz-Gene für bestimmte Antibiotika, insbesondere für Ampicillin und Tetracyclin.

8. Hefe, transformiert durch einen Plasmidvektor nach einem der Ansprüche 1 bis 7.

9. Hefe nach Anspruch 8, dadurch gekennzeichnet, daß sie dem Stamm Saccharomyces angehört.

10. Hefe nach Anspruch 9, dadurch gekennzeichnet, daß es sich um <u>Saccharomyces cerevisiae</u> handelt.

11. Verfahren zur Herstellung eines Proteins, dadurch gekennzeichnet, daß es darin besteht, ein Medium mit einer Hefe nach einem der Ansprüche 8 bis 10 zu fermentieren und das erzeugte Protein zu isolieren.

12. Verfahren nach Anspruch 11, dadurch gekennzeichnet, daß das erzeugte Protein β-Glucosidase ist.

13. β-Glucosidase, erhalten durch Durchführung des Verfahrens nach Anspruch 11, mit einem Plasmidvektor nach Anspruch 3.

## Claims

1. A plasmid vector for cloning and expression of a protein in a yeast, characterised in that it comprises at least the structural gene which codes for synthesis of the said protein and the components for expressing the structural gene in the yeast, and in that the structural gene is promoted by the expression promotor of the gene of β-glucosidase, the said promotor consisting in an ADN chromosome sequence extracted from a strain of <u>Kluyveromyces fragilis</u> and consisting of all or part of the BamH1 - BamH1 2.2 Kb restriction fragment.

2. A plasmid vector according to claim 1, characterised in that the structural gene codes for synthesis of β-glucosidase.

3. A plasmid vector according to claims 1 and 2, characterised in that the structural gene is the gene of <u>Kluyveromyces fragilis</u> which codes for synthesis of β-glucosidase.

4. A plasmid vector according to any of claims 1 to 3, characterised in that it also comprises:
- an origin of replication in yeasts, and
- a gene bringing about selection in yeasts.

5. A plasmid vector according to claim 4, characterised in that the origin of replication in yeasts is the origin of replication of 2 μ plasmid.

6. A plasmid vector according to claim 4, characterised in that the gene for bringing about selection is the gene ura3+.

7. A plasmid vector according to any of claims 1 to 6, characterised in that it also comprises:
- an origin of replication in bacteria, e.g. in <u>Escherichia Coli,</u> and
- genes for resistance to certain antibiotics, inter alia ampicillin and tetracycline.

8. A yeast transformed by a plasmid vector according to any of claims 1 to 7.

9. A yeast according to claim 8, characterised in that it belongs to the genus Saccharomyces.

10. A yeast according to claim 9, characterised in that it is <u>Saccharomyces cerevisiae.</u>

11. A method of producing a protein characterised in that it consists in fermenting a medium by using a yeast according to any of claims 8 to 10, and in isolating the resulting protein.

12. A method according to claim 11, characterised in that the resulting protein is β-glucosidase.

13. β-glucosidase obtained by working the method according to claim 11, using the plasmid vector in claim 3.

KF 1

## FIG.1

B: BamHI, Hp: HpaI, Pv: PvuII, S: SalI

## FIG_2

A : Ava I

H : Hind III

Hp : Hpa I

B : Bam HI

Bg : Bgl II

P : Pst I

## FIG_3

3